# EUROPEAN PATENT SPECIFICATION

(11) **EP 2 672 924 B1**
(45) Date of publication and mention of the grant of the patent: **20.12.2023**
(21) Application number: 12708146.1
(22) Date of filing: 08.02.2012
(51) Int. Cl.: A61F 2/00, D04B 1/00

(54) **VERY LIGHTWEIGHT SURGICAL MESH FOR VAGINAL PROLAPSE REPAIR**
SEHR LEICHTES CHIRURGISCHES NETZ ZUR SCHEIDENPROLAPSBEHEBUNG
MAILLE CHIRURGICALE ULTRALÉGÈRE CONÇUE POUR RÉPARER LE PROLAPSUS VAGINAL

(30) Priority: 08.02.2011 GB 201102165; 01.03.2011 GB 201103484
(43) Date of publication of application: 18.12.2013
(73) Proprietor: Atalla, Rami, Welwyn, Hertfordshire AL6 9RQ (GB)
(72) Inventor: Atalla, Rami, Welwyn, Hertfordshire AL6 9RQ (GB)
(74) Representative: Jacob, Reuben Ellis
(86) International application number: PCT/GB2012/000129
(87) International publication number: WO 2012/107722

(56) References cited:
- EP-A1- 1 520 552
- WO-A1-01/56499
- WO-A2-2009/156866
- US-A1- 2005 228 408
- US-A1- 2009 187 197

## Description

### Field of the Invention

The present invention relates to the field of surgical devices for vaginal prolapse repair, and in particular, to surgical meshes for use in surgical procedures for such vaginal repairs.

### Background of the invention

Prolapse of an internal organ through the vagina (e.g., cystocele, urethrocele, rectocele, enterocele, prolapsed uterus, vaginal vault prolapse) usually presents as a bulge in the vaginal walls. This often requires a surgical intervention in order to repair the vaginal prolapse, restore the prolapsed organ to its original position and maintain the vaginal walls in its anatomical position.

Surgeons use different techniques for repairing vaginal prolapse. The most common technique is the colporrhaphy. For example, for a cystocele, a vaginal wall is cut and separated from the bladder. The bladder is then pushed back in place and the pelvic floor tissues and fascia which have given way are stitched back to support the bladder.

Although this approach is widely used, the rate of recurrence when performing such operations is quite high. One of the reasons is the weakness of the pelvic floor and fascia, already damaged by the prolapsed organ. An alternative technique is the "site specific repair" used mainly in paravaginal defect repair, which involves re-approximating detached lateral vaginal tissue. Another technique involves the use of a prosthetic material to aid in support of the anterior or posterior vaginal walls, for example by using meshes or tension-free meshes.

A large variety of surgical meshes are now used by surgeons. They are usually classified by weight (heavyweight or lightweight) and by the size of the pores, i.e. the area between the fibres which form the meshes. In particular, small pore meshes have a pore diameter in the range of hundreds of microns (e.g., Marlex^{®} produced by Bard Inc.) whereas large pore meshes have a pore diameter in the range of a few millimetres (e.g., Vypro^{®} produced by Ethicon GmbH). Lightweight meshes usually have lower tensile strength than heavyweight meshes, whereas small pore meshes have less elasticity than large pore meshes. In general, lightweight meshes have less erosion and complications.

Surgical meshes for pelvic floor repair cause, however, a certain number of problems, such as foreign body reaction (FBR), vaginal mesh extrusion or exposure, pain (especially in the vagina during post-operation intercourse, the sensation of the presence of a mesh for both partners), infection, voiding dysfunction, recurrent prolapse and incontinence, shrinkage, chronic pain, infections, fistula and adhesion formation, calcification, degradation and erosion. Sometimes, such problems require complete removal of the surgical mesh originally inserted. This creates complications and discomfort in the affected patient. Such problems have even led certain health agencies to issue warnings about the use of the meshes currently on the market.

EP 1 520 552 relates to an implantable surgical mesh containing absorbable and non-absorbable portions.

Accordingly, there is a need for a medical device which could assist in repairing a vaginal prolapse and minimize the problems caused by the currently available meshes.

### Statement of the Invention

According to one aspect of the present invention, there is provided a surgical mesh for use in a surgical procedure for vaginal prolapse repair, the mesh including a first fibre or yarn and a second fibre or yarn, wherein the surgical mesh is so that the first fibre or yarn is free to slide over the second fibre or yarn, wherein the surgical mesh has a porosity at rest of about 70% or more wherein the average diameter of a pore at rest may be about 1 centimetre or more,

According to a second aspect of the invention, there is provided a process for knitting a surgical mesh for use in a surgical procedure for vaginal prolapse repair, the process including interweaving a first fibre or yarn with a second fibre or yarn so that the first fibre or yarn is free to slide over the second fibre or yarn, wherein the surgical mesh has a porosity at rest of 70% or more wherein the average diameter of a pore at rest is 1 centimetre or more.

### Brief Description of the Drawings

There now follows, by way of example only, a detailed description of embodiments of the present invention, with reference to the figures identified below.
Figure 1 shows an example of a cystocele as described in the background of the invention;
Figure 2 shows a surgical mesh in accordance with an embodiment of the present invention;
Figure 3 shows a close-up view of a surgical mesh in accordance with another embodiment of the present invention;
Figure 4 A and B show a surgical mesh formed from a monofilament in accordance with an embodiment of the present invention; and
Figure 5 shows some examples of possible fixing arrangements in accordance with an embodiment of the present invention.

### Detailed Description of the Embodiments

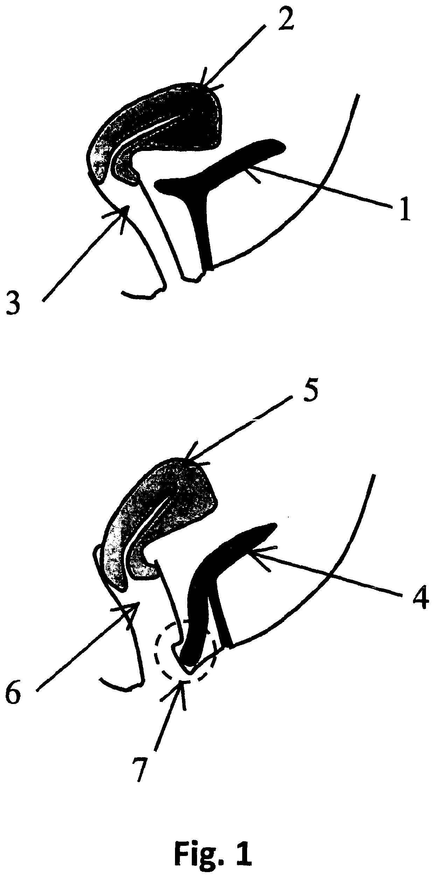

Figure 1 shows an example of cystocele as discussed in the background of the invention. The top of Figure 1 shows a normal situation, wherein the bladder (1) is in an anterior position with respect to the vagina (3), and the uterus (2) is in its normal position. The bottom of Figure 1, instead, shows a situation in which a cystocele has occurred. The bladder (4) has prolapsed into the vagina (6) creating a bulge (7). In addition, the uterus (5) has moved downwards into the vagina (6). Figure 1 only shows one of the possible conditions for which the medical device described in the present application is intended to be used. Other conditions include, for example, the conditions mentioned in the background to the invention in repair of vaginal prolapse.

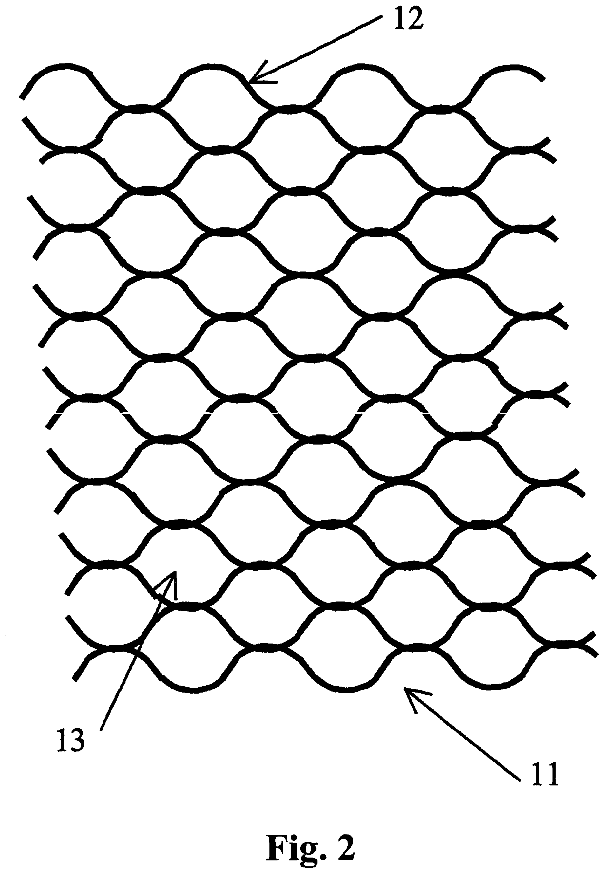

Figure 2 shows a medical device in accordance with an embodiment of the present invention. The medical device is a surgical mesh (11) with a porosity, at rest, of about 70% or more. Preferably, the porosity is strictly greater than 75%. "Porosity" is defined as the area in the plane of the mesh not blocked by the fibres of the mesh. "At rest" is intended when the medical device is not implanted in the body and/or no forces (e.g., pulling or pushing forces, tearing forces, stresses, gravity) are applied to it.

The mesh is formed of fibres or yarns. The fibres (12) can be monofilament or multifilament. A yarn can be produced by interlocking two or more of said fibres. Each fibre is made of polypropylene (PP) or any other non-absorbable or absorbable material suitable for its use.

At rest, the average diameter of a pore (13) is about 1 centimetre or more. At rest, the pores can have a diamond shape, a square shape, a circular shape, any polygonal shape or any irregular shape acquired by the process of interweaving (or knitting) the fibres or yarns.

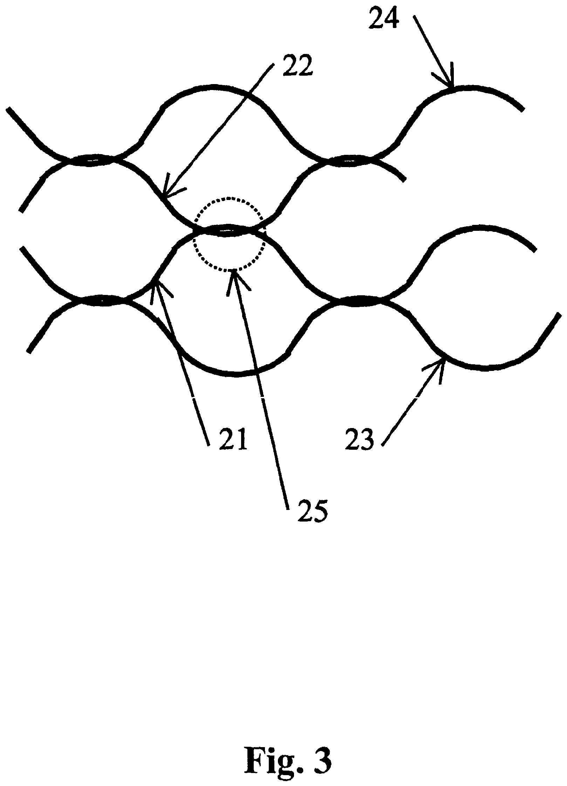

The mesh can be made by a knitting process. In particular, a first knitting process includes interweaving two or more fibres or yarns so that they are able to slide one over the other. An example of a resulting mesh is shown in Figure 3, wherein fibre or yarn (21) is able to move across or over fibre or yarn (22), and/or fibre or yarn (22) is able to move across or over fibre or yarn (21), for example around the intersection area (25). A similar mechanism occurs between fibres or yarns (21) and (23), or between fibres or yarns (22) and (24). The direction of the weaving can vary between one row and another.

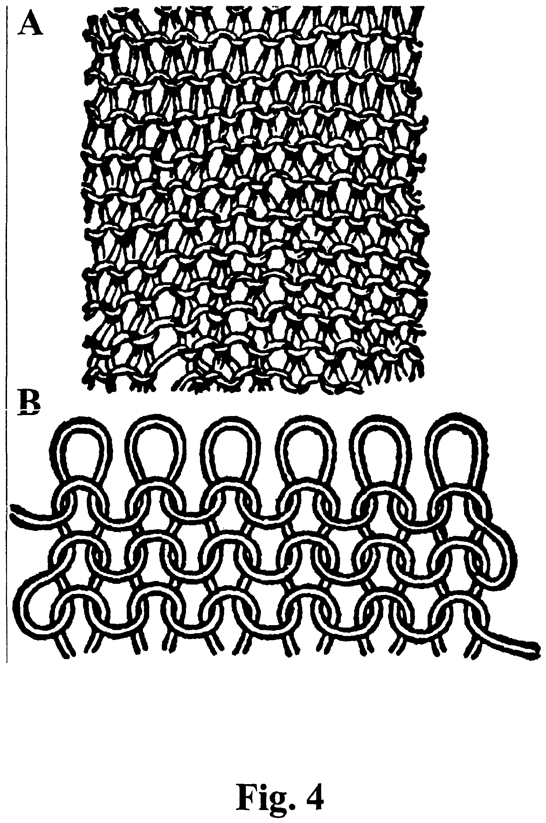

Figure 4 A and B show the another knitted pattern of the described surgical mesh formed from a monofilament. As can be seen from the mesh in Figure 4A, the surgical mesh is composed of a single fibre or yarn which is knitted to form pores of the features as described above. A closer view of the pattern of the knitted monofilament can be seen in Figure 4B.

In addition, fibre or yarn (21) and fibre or yarn (22) may be joined by a fixing arrangement around the intersection area (25). A fixing arrangement may limit the ability of fibre or yarn (21) and fibre or yarn (22) to slide over one another.

In an alternative embodiment, a second knitting process includes interweaving two or more fibres or yarns so that the first fibre or yarn is joined to the second fibre or yarn by a fixing arrangement or knot. The fixing arrangement may constrain a relative movement between a first fibre or yarn and a second fibre or yarn. The fixing arrangement may limit or block a relative movement between a first fibre or yarn and a second fibre or yarn.

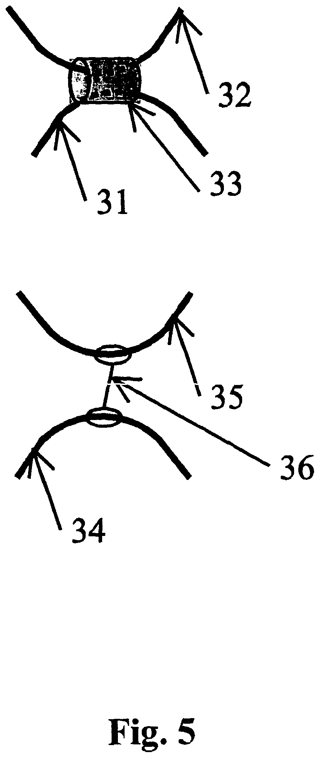

The fixing arrangement can be a simple knot of fibre or yarn (21) and fibre or yarn (22), a fastening element or a securing element. Figure 5 shows some non-limiting examples of possible fixing arrangements. The fixing arrangement may be an element (33) that surrounds fibres or yarns (31) and (32) at the intersection area. This may allow fibres or yarns (31) and (32) (or, equivalently fibres or yarns (21) and (22) of Figure 3) to be free to move with respect to one another without crossing over at the intersection area. It may also be used to secure, limit or block the movement of fibres or yarns (31) and (32) when they are crossed over at the intersection area. The fixing arrangement may also be a bridging element (36) that joins fibre or yarn (34) and fibre or yarn (35).

The device described above presents many advantages over the surgical meshes currently in use.

The mesh resulting from the first knitting process described above presents the advantage that the movement of each fibre or yarn with respect to one another adds flexibility to the mesh, and consequently advantageously improves the elasticity of the mesh (some of the advantages of an increased elasticity are discussed below).

In addition, each fibre or yarn is allowed to extend along its axis, thus increasing the stretchability of the mesh, which in turn is able to contain in a more efficient way any weight or pressure applied to it, such as a prolapsed organ, a vaginal wall or any object pressing on it. The mesh structure can contribute in guaranteeing that the fibres are not overstretched so as to reach their yield point. In this way, the overall chance of rupture or erosion of the mesh is also reduced.

The same can also be true for a mesh resulting from the second knitting process when the knot arrangement constrains without blocking a relative movement between a first fibre or yarn and a second fibre or yarn.

FBR depends on the surface area in contact with the host tissue, and the surface area strongly depends on textile properties such as the pore size or diameter and number of fibres/yarns used. A mesh in accordance with the invention reduces the surface area by a significant factor. Porosity, at rest, of about 70% or more (preferably strictly greater than 75%) and an average diameter of a pore (3), at rest, of about 1 centimetre or more imply that the surface area in contact with the host tissue is minimised. Moreover, larger pores will help in the healing process of the tissues as they allow more body tissue contact and hence faster healing.

This also benefits the healing process, as tissues can grow in a more efficient way. It thus reduces or eliminates the need of material and/or substance to help the healing processes, which is often required in meshes with a smaller porosity. Moreover, fibrotic reactions resulting from inflammatory response around the mesh is greatly reduced by the presence of minimal body (i.e., the material of the fibre).

Moreover, the decrease in weight of the mesh in accordance with the present invention, and hence the amount of foreign body involved in the repair, reduces the chance of rejection or exposure of the mesh.

The stiffness of the mesh is also greatly reduced. Tissue growth around the mesh causes the structure to lose elasticity because of the fibrotic bridgings created between the fibres/yarns. As a consequence, the mesh becomes more rigid, and is more likely to cause discomfort for one or both partners during intercourse. The surgical mesh provided by this invention considerably reduces the risk of losing elasticity, because the fibres are spaced apart from each other, thus reducing the occurrence of fibrotic bridging.

In addition, the structure of the mesh guarantees increased elasticity and reduces problems which are usually experienced during post-operation sexual intercourse. When incorporated in the pelvic floor, a mesh structure acts as a reinforcement for the vaginal walls. This also has a consequence on the vaginal wall, which is able to sustain a greater pressure. However, when the mesh loses elasticity, the vaginal wall becomes less flexible. This can cause problems during intercourse when pressure is applied on the vaginal wall. Besides, as the thickness of the wall is relatively low, the stiff mesh can be felt, and there is a greater risk of vaginal exposure. The mesh structure in accordance with the invention greatly reduces these problems, since the elevated porosity guarantees greater elasticity and reduced stiffness, as described above. Thus, the reinforced vaginal wall will be more flexible to pressure, and at the same time the risk of exposing the mesh will be greatly reduced.

Moreover, all the above advantages are further achieved by the combination of the large porosity of the mesh and the freedom of movement of the fibres or yarns in the mesh guaranteed by the knitting process described above.

The overall shape at rest of the mesh will vary depending on the site of insertion. For instance, the shape of the mesh for the anterior vaginal wall will differ from the shape of the mesh for the posterior vaginal wall or from the shape of the mesh for the vault prolapse. The mesh may be provided with arms for ease of insertion. The arms may be reinforced at the angles of the arms.

The mesh have a knitted borders to maintain the knitted mesh in its format and avoid the fibres being displaced. The knitted borders also help to preserve the shape of the mesh during insertion in the operation and distribute the pulling on the mesh fibres.

The medical device described above can be used in any surgical procedure for repairing vaginal prolapse, such as anterior or posterior vaginal wall repair and vaginal vault prolapse repair.

## Claims

1. A surgical mesh (11) for use in a surgical procedure for vaginal prolapse repair, the mesh (11) including a first fibre or yarn (21), (31), (34) and a second fibre or yarn (22), (32), (35), wherein the surgical mesh (11) is knitted so that the first fibre or yarn (21), (31), (34) is freely able to slide over the second fibre or yarn (22), (32), (35), wherein the surgical mesh (11) has a porosity at rest of 70% or more wherein the average diameter of a pore at rest is 1 centimetre or more.

2. The surgical mesh (11) of claim 1, the surgical mesh (11) having a porosity at rest strictly greater than 75%.

3. The surgical mesh (11) of claim 2, the surgical mesh (11) having a porosity at rest strictly greater than 90%.

4. The surgical mesh of claim 1, wherein the average diameter of a pore at rest is 1.1 centimetre or more.

5. The surgical mesh (11) of any one of one of the above claims, wherein a pore has a diamond shape, a square shape, a circular shape, any polygonal shape or any irregular shape acquired by the knitting of the fibres.

6. The surgical mesh (11) of any one of the above claims, further including arms for ease of insertion of the surgical mesh (11) during the surgical procedure.

7. The surgical mesh (11) of any of the above claim, wherein the surgical mesh (11) has a knitted border.

8. The surgical mesh (11) of any of the above claims, wherein the mesh (11) further comprises absorbable or non-absorbable fibre or yarn.

9. A process for knitting a surgical mesh (11) for use in a surgical procedure for vaginal prolapse repair, the process including interweaving a first fibre or yarn (21), (31), (34) with a second fibre or yarn (22), (32), (35) so that the first fibre or yarn (21), (31), (34) is able to slide over the second fibre or yarn (22), (32), (35), wherein the surgical mesh has a porosity at rest of 70% or more wherein the average diameter of a pore at rest is 1 centimetre or more.

10. The process of claim 9, wherein the interweaving is such that the second fibre or yarn (22), (32), (35) is able to slide over the first fibre or yarn (21), (31), (34).

## Patentansprüche

1. Chirurgisches Netz (11) zur Verwendung in einem chirurgischen Eingriff zur Scheidenprolapsbehebung, wobei das Netz (11) eine erste Faser oder ein erstes Garn (21), (31), (34) und eine zweite Faser oder ein zweites Garn (22), (32), (35) umfasst, wobei das chirurgische Netz (11) gestrickt ist, sodass die erste Faser oder das erste Garn (21), (31), (34) ungehindert über die zweite Faser oder das zweite Garn (22), (32), (35) gleiten kann, wobei das chirurgische Netz (11) eine Porosität im Ruhezustand von 70 % oder mehr aufweist, wobei der Durchschnittsdurchmesser einer Pore im Ruhezustand 1 Zentimeter oder mehr beträgt.

2. Chirurgisches Netz (11) nach Anspruch 1, wobei das chirurgische Netz (11) eine Porosität im Ruhezustand strikt größer als 75 % aufweist.

3. Chirurgisches Netz (11) nach Anspruch 2, wobei das chirurgische Netz (11) eine Porosität im Ruhezustand strikt größer als 90 % aufweist.

4. Chirurgisches Netz nach Anspruch 1, wobei der Durchschnittsdurchmesser einer Pore im Ruhezustand 1,1 Zentimeter oder mehr beträgt.

5. Chirurgisches Netz (11) nach einem der vorangehenden Ansprüche, wobei eine Pore eine Rautenform, eine Quadratform eine Kreisform, eine beliebige vieleckige Form oder eine beliebige durch das Stricken der Fasern erhaltende unregelmäßige Form aufweist.

6. Chirurgisches Netz (11) nach einem der vorangehenden Ansprüche, ferner umfassend Arme zum leichten Einbringen des chirurgischen Netzes (11) während des chirurgischen Eingriffs.

7. Chirurgisches Netz (11) nach einem der vorangehenden Ansprüche, wobei das chirurgische Netz (11) einen gestrickten Rand aufweist.

8. Chirurgisches Netz (11) nach einem der vorangehenden Ansprüche, wobei das Netz (11) ferner eine absorbierbare oder nicht absorbierbare Faser oder ein absorbierbares oder nicht absorbierbares Garn umfasst.

9. Verfahren zum Stricken eines chirurgischen Netzes (11) zur Verwendung in einem chirurgischen Eingriff zur Scheidenprolapsbehebung, wobei das Verfahren das Verflechten einer ersten Faser oder eines ersten Garns (21), (31), (34) mit einer zweiten Faser oder einem zweiten Garn (22), (32), (35) umfasst, sodass die erste Faser oder das erste Garn (21), (31), (34) über die zweite Faser oder das zweite Garn (22), (32), (35) gleiten kann, wobei das chirurgische Netz (11) eine Porosität im Ruhezustand von 70 % oder mehr aufweist, wobei der Durchschnittsdurchmesser einer Pore im Ruhezustand 1 Zentimeter oder mehr beträgt.

10. Verfahren nach Anspruch 9, wobei das Verflechten derart ist, dass die zweite Faser oder das zweite Garn (22), (32), (35) über die erste Faser oder das erste Garn (21), (31), (34) gleiten kann.

## Revendications

1. Filet chirurgical (11) destiné à être utilisé dans le cadre d'une intervention chirurgicale à des fins de réparation suite à un prolapsus vaginal, le filet (11) comprenant une première fibre ou un premier fil (21), (31), (34) et une deuxième fibre ou un deuxième fil (22), (32), (35), dans lequel le filet chirurgical (11) est tricoté de telle sorte que la première fibre ou le premier fil (21), (31), (34) est librement en mesure de glisser par-dessus la deuxième fibre ou le deuxième fil (22), (32), (35), dans lequel le filet chirurgical (11) a une porosité au repos de 70 % ou plus, dans lequel le diamètre moyen d'un pore au repos est de 1 centimètre ou plus.

2. Filet chirurgical (11) selon la revendication 1, le filet chirurgical (11) ayant une porosité au repos strictement supérieure à 75 %.

3. Filet chirurgical (11) selon la revendication 2, le filet chirurgical (11) ayant une porosité au repos strictement supérieure à 90 %.

4. Filet chirurgical selon la revendication 1, dans lequel le diamètre moyen d'un pore au repos est de 1,1 centimètre ou plus.

5. Filet chirurgical (11) selon l'une quelconque des revendications ci-dessus, dans lequel un pore a une forme de losange, une forme carrée, une forme circulaire, toute forme polygonale ou toute forme irrégulière acquise par le tricotage des fibres.

6. Filet chirurgical (11) selon l'une quelconque des revendications ci-dessus, comprenant par ailleurs des bras servant à faciliter l'insertion du filet chirurgical (11) au cours de l'intervention chirurgicale.

7. Filet chirurgical (11) selon l'une quelconque des revendications ci-dessus, dans lequel le filet chirurgical (11) a une bordure tricotée.

8. Filet chirurgical (11) selon l'une quelconque des revendications ci-dessus, dans lequel le filet (11) comporte par ailleurs une fibre ou un fil absorbable ou non absorbable.

9. Procédé servant à tricoter un filet chirurgical (11) destiné à être utilisé dans le cadre d'une intervention chirurgicale à des fins de réparation suite à un prolapsus vaginal, le procédé comprenant l'étape consistant à entrelacer une première fibre ou un premier fil (21), (31), (34) avec une deuxième fibre ou un deuxième fil (22), (32), (35) de telle sorte que la première fibre ou le premier fil (21), (31), (34) est en mesure de glisser par-dessus la deuxième fibre ou le deuxième fil (22), (32), (35), dans lequel le filet chirurgical a une porosité au repos de 70 % ou plus, dans lequel le diamètre moyen d'un pore au repos est de 1 centimètre ou plus.

10. Procédé selon la revendication 9, dans lequel l'entrelacement est tel que la deuxième fibre ou le deuxième fil (22), (32), (35) est en mesure de glisser par-dessus la première fibre ou le premier fil (21), (31), (34).
